(19) Europäisches Patentamt
European Patent Office
Office européen des brevets

(11) Veröffentlichungsnummer: **0 173 932**
**A2**

(12) # EUROPÄISCHE PATENTANMELDUNG

(21) Anmeldenummer: **85110640.1**

(22) Anmeldetag: **23.08.85**

(51) Int. Cl.[4]: **A 61 K 7/13**
C 07 C 87/62, C 07 C 91/32

(30) Priorität: **01.09.84 DE 3432214**

(43) Veröffentlichungstag der Anmeldung:
**12.03.86 Patentblatt 86/11**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Rose, David, Dr.**
**Holbeinweg 7**
**D-4010 Hilden(DE)**

(72) Erfinder: **Lieske, Edgar**
**Hunsrückenstrasse 40**
**D-4000 Düsseldorf(DE)**

(72) Erfinder: **Maak, Norbert, Dr.**
**Im Jagdfeld 41a**
**D-4040 Neuss(DE)**

(54) **Haarfärbemittel.**

(57) N-Benzyl-p-phenylendiamin und Derivate der allgemeinen Formel

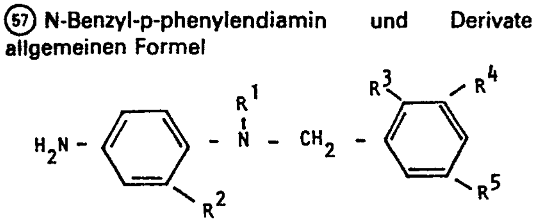

(I)

in der $R^1$ Wasserstoff oder eine alkylgruppe mit 1 bis 4 C-Atomen, $R^2$ Wasserstoff, Chlor oder eine Alkylgruppe mit 1 bis 4 C-Atomen, $R^3$ Wasserstoff oder eine Hydroxylgruppe und $R^4$ und $R^5$ unabhängig voneinander Wasserstoff oder Chlor bedeuten, oder deren Salze eignen sich als Oxidationshaarfarbstoffvorprodukte für Haarfärbemittel. Die Verbindungen ergeben allein oder zusammen mit bekannten Kupplern und/oder Entwicklerverbindungen brillante Haarfärbungen von hoher Echtheit – vornehmlich im Bereich blauer, roter und brauner Nuancen.

EP 0 173 932 A2

Croydon Printing Company Ltd.

0173932

HENKEL KGaA
ZR-FE/Patente

4000 Düsseldorf, den 25. Juni 1985
Henkelstraße 67

Dr. JG/Sr

Patentanmeldung

D 7087 EP

## Haarfärbemittel

Gegenstand der Erfindung sind Haarfärbemittel auf der Basis von Oxidationsfarbstoffen. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt, die unter dem Einfluß von Oxidationsmitteln oder von Luftsauerstoff Farbstoffe ausbilden. Als kosmetische Träger für die Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, hetero-cyclische Hydrazonderivate, 4-Aminopyrazolonderivate und Tetraaminopyrimidine eingesetzt. Als sogenannte Kupplersubstanzen werden m-Phenylendiaminderivate, Naphthole, Resorcinderivate und Pyrazolone verwendet.

...

Gute Oxidationshaarfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf menschlichem Haar besitzen ohne die Kopfhaut zu stark anzufärben und sie sollen vor allem in toxikologischer und dermatologischer Hinsicht unbedenklich sein.

P-Phenylendiamin und viele Derivate dieser Verbindung sind zwar als Oxidationsfarbstoffvorprodukte seit langem bekannt. Es ist aber auch bekannt, daß viele dieser Verbindungen toxikologisch und dermatologisch bedenklich sind, da sie mutagene und/oder allergisierende Eigenschaften aufweisen. Es besteht daher ein hohes technisches Interesse an Oxidationsfarbstoffvorprodukten, die einerseits intensive Farben mit hohen Echtheitseigenschaften ausbilden, andererseits aber in toxikologischer und dermatologischer Hinsicht sicher sind.

Es wurde nun gefunden, daß Haarfärbemittel, enthaltend Oxidationsfarbstoffvorprodukte in einem kosmetischen Träger, die als Oxidationsfarbstoffvorprodukte N-Benzyl-p-phenylendiamin oder dessen Derivate der allgemeinen Formel

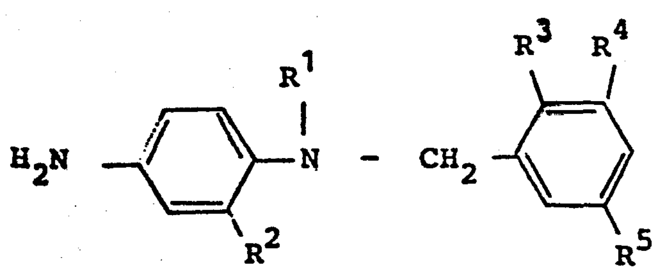

in der $R^1$ Wasserstoff oder eine Alkylgruppe mit 1 - 4 C-Atomen, $R^2$ Wasserstoff, Chlor- oder eine Alkylgruppe mit 1 - 4 C-Atomen, $R^3$ Wasserstoff oder eine Hydroxylgruppe und $R^4$ und $R^5$ unabhängig voneinander Wasserstoff oder Chlor bedeuten oder deren Salze und die in Oxidationsfärbemitteln üblichen Kuppler- und/oder Entwicklersubstanzen enthalten, die gestellten Anforderungen in

...

hohem Maße erfüllen und vor allem in bezug auf die toxikologischen und dermatologischen Eigenschaften unbedenklich und sicher sind. Die erfindungsgemäßen Haarfärbemittel liefern besonders intensive und brillante Haarfärbungen von hoher Licht- und Wärmeechtheit, vornehmlich im Bereich der blauen, roten und braunen Nuancen.

Besonders intensive und stabile Farbnuancen werden erhalten, wenn die erfindungsgemäß zu verwendenden N-Benzyl-p-phenylendiamine der Formel I die Substituenten in den Kombinationen E1, E2, E3, E4 oder E5 enthalten.

| | | | | | |
|---|---|---|---|---|---|
| E 1: | $R^1$ = H | $R^2$ = H | $R^3$ = H | $R^4$ = H | $R^5$ = H |
| E 2: | $R^1$ = $CH_3$ | $R^2$ = H | $R^3$ = H | $R^4$ = H | $R^5$ = H |
| E 3: | $R^1$ = H | $R^2$ = Cl | $R^3$ = H | $R^4$ = H | $R^5$ = H |
| E 4: | $R^1$ = H | $R^2$ = H | $R^3$ = OH | $R^4$ = Cl | $R^5$ = Cl |
| E 5: | $R^1$ = H | $R^2$ = $CH_3$ | $R^3$ = OH | $R^4$ = H | $R^5$ = H |

Das erfindungsgemäß als Oxidationshaarfarbstoffvorprodukt zu verwendende N-Benzyl-p-phenylendiamin (E1) ist eine literaturbekannte Verbindung (vgl. Annalen der Chemie, 263, 302). Die übrigen der allgemeinen Formel I entsprechenden Verbindungen sind nach literaturbekannten Syntheseverfahren zugänglich. Allgemein kann man, wenn $R^3$, $R^4$ und $R^5$ Halogen oder eine Hydroxylgruppe bedeuten, ein entsprechend substituiertes p-Nitroanilin mit einem entsprechend substituiertem Benzylalkohol nach dem allgemeinen Reaktionsschema

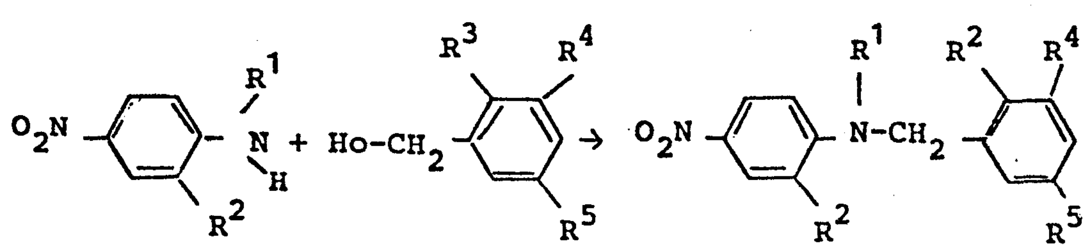

oder, wenn $R^3$, $R^4$ und $R^5$ Wasserstoff oder Alkylgruppen sind, ein entsprechend substituiertes p-Chlor-nitrobenzol mit einem

...

entsprechend substituierten Benzylamin nach dem allgemeinen Reaktionsschema

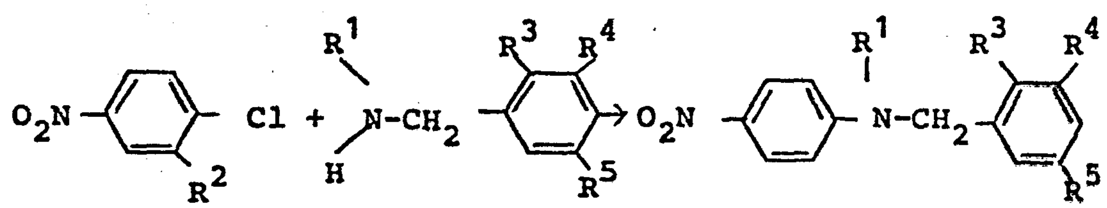

zum N-Benzyl-p-Nitroanilin umsetzen und dieses katalytisch zum N-Benzyl-p-phenylendiamin hydrieren. Die Herstellung der in der Literatur nicht bekannten Derivate E 2 - E 5 wird in den Beispielen näher beschrieben.

Die erfindungsgemäß zu verwendenden N-Benzyl-p-phenylendiamine der allgemeinen Formel I sind Entwicklersubstanzen, d.h. sie vermögen unter Einwirkung von Oxidationsmitteln Farbstoffe auszubilden. In Gegenwart von Kupplersubstanzen werden jedoch besonders brillante und intensive Farben gebildet. Als Kupplersubstanzen eignen sich vor allem die m-Phenylendiamine, m-Aminophenole, Resorcine, Naphthol-1, 1,5- und 2,7-Dihydroxynaphthalin, Hydroxy- und Aminopyridine und Aminopyrazolone. Zur Modifikation der Farbnuance sind jedoch nicht nur die typischen, literaturbekannten Kupplerverbindungen geeignet, die selbst (ohne Entwickler) keine brauchbaren Färbungen anzubilden vermögen, sondern auch typische Entwicklersubstanzen, die mit den erfindungsgemäß zu verwendenden N-Benzyl-p-phenylendiaminen zu neuen Nuancen kuppeln. Solche zur Modifikation der Haaranfärbung geeignete Verbindungen sind vor allem aromatische Amine, die in Ortho- oder Paraposition eine zweite, substituierte oder freie Aminogruppe, eine Hydroxylgruppe, eine Phenolethergruppe oder eine Thiophenolethergruppe tragen, Diaminopyridine, 4-Aminopyrazolone sowie 2.4.5.6.-Tetraaminopyrimidin und deren Derivate. Auch direktziehende Farbstoffe, z.B.

...

Nitrophenylendiaminderivate, Anthrachinonfarbstoffe oder Indophenole können zur Herstellung natürlicher Haarfarbnuancen mit den Oxidationsfarbstoffvorprodukten kombiniert und in die erfindungsgemäßen Haarfärbemittel eingesetzt werden.

In den erfindungsgemäßen Haarfärbemitteln werden die Entwicklerverbindungen und die Kupplerverbindungen im allgemeinen in etwa äquimolaren Mengen eingesetzt. Wenn sich auch der äquimolare Einsatz als zweckmäßig erweist, so ist es jedoch nicht nachteilig, wenn einzelne Oxidationsfarbstoffvorprodukte im Überschuß zum Einsatz gelangen. Es ist auch nicht erforderlich, daß die N-Benzyl-p-Phenylendiamine oder die sonst vorhandenen Oxidationsfarbstoffvorprodukte einheitliche Verbindungen sind. Vielmehr können auch Gemische dieser Verbindungen zum Einsatz gelangen.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zur Herstellung der erfindungsgemäßen Haarfärbemittel werden die Oxidationsfarbstoffvorprodukte in einen geeigneten kosmetischen Träger eingearbeitet. Solche Träger sind zum Beispiel Cremes, Emulsionen, Gele oder auch tensidhaltige, schäumende Lösungen, zum Beispiel Shampoos oder andere Zubereitungen, die für die Anwendung auf dem Haar geeignet sind. Übliche Bestandteile solcher kosmetischer Zubereitungen sind zum Beispiel Netz- und Emulgiermittel wie anionische, nichtionische oder ampholytische Tenside, zum Beispiel Fettalkoholsulfate, Alkansulfonate, $\alpha$-Olefinsulfonate, Fettalkoholpolyglykolethersulfate, Ethylenoxidanlagerungsprodukte an Fettalkohole, Fettsäuren und Alkylphenole,

...

Sorbitanfettsäureester und Fettsäurepartialglyceride, Fettsäurealkanolamide sowie Verdickungsmittel wie z.B. Methyl- oder Hydroxyethylcellulose, Stärke, Fettalkohole, Paraffinöle, Fettsäuren, ferner Parfümöle und haarpflegende Zusätze, wie z.B. wasserlösliche kationische Polymere, Proteinderivate, Pantothensäure und Cholesterin. Die Bestandteile der kosmetischen Träger werden zur Herstellung der erfindungsgemäßen Haarfärbemittel in für diese Zwecke üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt. Die Oxidationsfarbstoffvorprodukte werden in Mengen von 0,2 bis 5 Gew.-%, vorzugsweise 1 bis 3 Gew.-% des gesamten Färbemittels in den Träger eingemischt. Die erfindungsgemäß zu verwendenden N-Benzyl-p-phenylendiamine können in einer Menge von 0,05 bis 10 Millimol pro 100 g eingesetzt werden.

Die Anwendung der erfindungsgemäßen Haarfärbemittel kann, unabhängig von der Art der kosmetischen Zubereitung, z.B. als Creme, Gel oder Shampoo, im schwach sauren, neutralen oder alkalischen Milieu erfolgen. Bevorzugt ist die Anwendung der Haarfärbemittel in einem pH-Bereich von 8 - 10. Die Anwendungstemperaturen können in einem Bereich zwischen 15°C und 40°C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z.B. ein Färbeshampoo, verwendet wurde.

Die mit den erfindungsgemäßen Haarfärbemitteln erzielbaren Färbungen haben eine hohe Brillanz sowie überlegene Wärme-, Licht-, Wasch- und Reibechtheitseigenschaften. Die nachfolgenden Beispiele sollen den Erfindungsgegenstand näher erläutern, ohne ihn jedoch hierauf zu beschränken.

. . .

## Beispiele

### Herstellungsbeispiele

**1-Benzyl-methylamino-4-aminobenzol (E 2)**

1. Stufe: N-Benzyl-N-methyl-p-nitroanilin

Eine Mischung aus 7,9 g 1-Chlor-4-nitrobenzol (0,05 Mol) und 12,1 g Benzylmethylamin (0,1 Mol) wurde 6 Stunden unter Rühren auf 180°C erhitzt. Nach dem Abkühlen auf 20°C wurde das Reaktionsgemisch in verdünnter Salzsäure aufgenommen und der Niederschlag abfiltriert, mit Wasser gewaschen, getrocknet und aus Ethanol umkristallisiert. Es wurden 8,2 g eines gelben Pulvers mit einem Schmelzpunkt von 66°C erhalten.

2. Stufe: 1-Benzyl-methylamino-4-aminobenzol

Das Produkt der 1. Stufe wurde in ca. 200 ml Ethanol in Gegenwart von 0,5 g Palladium auf Aktivkohle (10 %ig) katalytisch hydriert. Nach beendeter Wasserstoffaufnahme wurde der Katalysator durch Filtration abgetrennt und das Filtrat zur Trockene eingeengt. Es wurde ein hellbraunes Öl erhalten und durch Infrarotspektrum identifiziert:

IR-Spektrum (Film): 1615, 1520, 1470, 1450, 1430, 1415, 1335, 1310, 1260, 1180, 1150, 1125, 1050, 820.

**1-Benzylamino-2-chlor-4-aminobenzol-dihydrochlorid (E 3)**

1. Stufe: 1-Benzylamino-2-chlor-4-nitrobenzol

Eine Mischung aus 9,6 g 3.4-Dichlor-nitrobenzol (0,05 Mol) und 16g Benzylamin (0,15 Mol) wurde 30 Minuten auf 110°C und

. . .

anschließend 2 Stunden auf 150° C erhitzt. Nach dem Abkühlen wurde das Reaktionsgemisch auf Eiswasser gegossen, der Niederschlag abfiltriert, mit Wasser gewaschen und aus Ethanol umkristallisiert. Es wurden 10,8 g eines gelben Pulvers mit einem Schmelzpunkt von 95° C erhalten.

2. Stufe: 1-Benzylamino-2-chlor-4-aminobenzol-dihydrochlorid

2,6 g des Produktes der 1. Stufe wurden mit 11,3 g $Sn\ Cl_2 \cdot 2H_2O$ in 20 ml Ethanol 40 Minuten zum Sieden unter Rückflußkühlung erhitzt. Nach dem Abkühlen wurden 100 ml Eiswasser zugesetzt und unter Rühren ein pH-Wert von ca. 8 eingestellt. Das Reaktionsgemisch wurde dann 2 x mit je 50 ml Ethylacetat extrahiert. Nach Einengen des Estraktes zur Trockene wurde der Rückstand mit ca. 5 ml konzentrierter Salzsäure versetzt und erneut zur Trockene eingeengt. Es wurden 1,6 g des Produktes mit einem Schmelzpunkt von 184° C (unter Zersetzung) erhalten.

<u>1-(2-Hydroxy-3.5-dichlorbenzyl)-amino-4-aminobenzoldihydrochlorid (E 4)</u>

1. Stufe: 1-(2-Hydroxy-3.5-dichlor)-benzylamino-4-nitrobenzol

Eine Mischung aus 9,65 g 2-Hydroxy-3.5-dichlorbenzylalkohol (0,05 Mol) und 6,9 g p-Nitroanilin (0,05 Mol) wurde 1 Stunde unter Rühren auf 160°C erhitzt. Nach dem Erkalten wurde das Reaktionsgemisch in verdünnter Natronlauge ( 45 ml 10 %ige Natronlauge in300 ml Wasser) aufgenommen. Durch Einleiten von $CO_2$-Gas wurde das Produkt ausgefällt, dann abfiltriert, getrocknet und aus Ethanol umkristallisiert. Es wurden 8 g eines gelben Pulvers mit einem Schmelzpunkt von 129 - 132°C erhalten.

2. Stufe: 1-(2-Hydroxy-3.5-dichlorbenzyl)-amino-4-aminobenzol-dihydrochlorid

. . .

10 g des Produktes der 1. Stufe wurden in 400 ml Ethanol gelöst und nach Zugabe von 0,5 g Raney-Nickel katalytisch hydriert. Nach beendeter Wasserstoff-Aufnahme wurde der Katalysator durch Filtration abgetrennt und das Filtrat mit konzentrierter Salzsäure angesäuert. Nach dem Einengen wurden 5 g des 1-(2-Hydroxy-3.5-dichlor)-benzylamino-4-aminobenzol-dihydrochlorid mit einem Schmelzpunkt ab 190°C (unter Zersetzung) erhalten.

1-(2-Hydroxybenzyl)-amino-2-methyl-4-aminobenzol-dihydrochlorid (E5)

1. Stufe: 1-(2-Hydroxybenzyl)-amino-2-methyl-4-nitrobenzol

Eine Mischung aus 12,4 g 2-Hydroxybenzylalkohol (0,1 Mol) und 15,2 g 2-Methyl-4-nitroanilin (0,1 Mol) wurde 2 Stunden unter Rühren auf 160 °C erhitzt. Nach dem Erkalten wurde das Reaktionsgemisch in verdünnter Natronlauge (45 ml 10%ige Natronlauge in 300 ml Wasser) aufgenommen. Durch Einleiten von $CO_2$-Gas wurde das Produkt ausgefällt, dann abfiltriert, getrocknet und aus Ethanol umkristallisiert. Es wurden 6 g eines olivgelben Pulvers mit einem Schmelzpunkt von 125 °C (unter Zersetzung) erhalten.

2. Stufe: 1-(2-Hydroxybenzyl)-amino-2-methyl-4-aminobenzol-dihydrochlorid

5 g des Produktes der 1. Stufe wurden in 400 ml Ethanol gelöst und nach Zugabe von 0,5 g Raney-Nickel katalytisch hydriert. Nach beendeter Wasserstoff-Aufnahme wurde der Katalysator durch Filtration abgetrennt und das Filtrat mit konzentrierter Salzsäure angesäuert. Nach dem Einengen wurden 4,2 g des 1-(2-Hydroxybenzyl)-amino-2-methyl-4-aminobenzol-dihydrochlorid mit einem Schmelzpunkt von 210 °C (unter Zersetzung) erhalten.

. . .

## Anwendungsbeispiele

Es wurden erfindungsgemäße Haarfärbemittel in Form einer Haarfärbe-Cremeemulsion der folgenden Zusammensetzung hergestellt:

| | |
|---|---|
| Fettalkohol $C_{12-18}$ | 10 g |
| Fettalkohol $C_{12-14}$ + 2 EO-sulfat, Na-Salz, 28 %ig | 25 g |
| Wasser | 60 g |
| N-Benzyl-p-phenylendiaminderivat (E) | 7,5 mMol |
| 2. Oxidationsfarbstoffvorprodukt (M) | 7,5 mMol |
| $Na_2SO_3$ (Inhibitor) | 1,0 g |
| konzentrierte Ammoniak-Lösung | bis pH = 9,5 |
| Wasser | ad 100 g |

Die Bestandteile wurden der Reihe nach miteinander vermischt. Nach Zugabe der Oxidationsfärbemittelvorprodukte und des Inhibitors wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3%iger Wasserstoffperoxidlösung als Oxidationsmittel durchgeführt. Hierzu wurden 100 g der Emulsion mit 50 g Wasserstoffperoxidlösung (3%ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 $^{0}$ C belassen. Nach Beendigung des Färbeprozesses wurde das Haar gespült, mit einem üblichen Haarwaschmittel ausgewaschen und anschließend getrocknet.

...

Es wurden folgende N-Benzyl-p-phenylendiamine eingesetzt (Komponente E)

E 1 : 1-Benzylamino-4-aminobenzol
E 2 : 1-Benzyl-methyl-amino-4-aminobenzol
E 3 : 1-Benzylamino-2-chlor-4-aminobenzol-dihydrochlorid
E 4 : 1-(2-Hydroxy-3.5-dichlorbenzyl)-amino-4-aminobenzol-dihydrochlorid
E 5 : 1-(2-Hydroxybenzyl)-amino-2-methyl-4-aminobenzol-dihydrochlorid

Als zweites Oxidationsfarbstoffvorprodukt (Komponente M) zur Modifikation der Haaranfärbung wurden folgende Verbindungen eingesetzt:

M 1 : Naphthol-1
M 2 : 1.5-Dihydroxynaphthalin
M 3 : 2.7-Dihydroxynaphthalin
M 4 : Resorcin
M 5 : 2-Methylresorcin
M 6 : 4-Chlorresorcin
M 7 : Diresorcylsulfoxid
M 8 : m-Aminophenol
M 9 : 5-Amino-2-methylphenol
M 10: 2.4-Dichlor-3-aminophenol
M 11: 2.4-Diaminophenol
M 12: 3.4-Methylendioxyphenol
M 13: 3.4-Methylendioxyanilin
M 14: 2.4-Diaminophenoxyethanol
M 15: 1.3-Bis-(2.4-diaminophenoxy)-propan
M 16: 5-Chlor-2.3-pyridindiol
M 17: 2-Amino-3-hydroxypyridin
M 18: 3-Amino-2-methylamino-6-methoxypyridin
M 19: 1-Phenyl-3-methyl-pyrazolon-5
M 20: 1-Phenyl-4-amino-pyrazolon-5

. . .

M 21: p-Phenylendiamin
M 22: 2.5-Diaminotoluol
M 23: N-Methyl-p-phenylendiamin
M 24: 1-(2-Hydroxypropyl)-amino-4-aminobenzol
M 25: 1-Ethyl-1-(2-hydroxyethyl)-amino-4-aminobenzol
M 26: 1.1-Bis-(2-hydroxyethyl)-amino-4-aminobenzol
M 27: 2.5-Diaminoanisol
M 28: p-Aminophenol
M 29: 2.4.5.6-Tetraaminopyrimidin

Die mit den genannten Oxidationsfärbemittelvorprodukten erhaltenen Haaranfärbungen sind der Tabelle I zu entnehmen:

Tabelle I

| Anwendungsbeispiel | Komponente E | Komponente M | erhaltener Farbton |
|---|---|---|---|
| A 1 | E 1 | M 1 | schwarzblau |
| A 2 | E 1 | M 2 | schwarzblau |
| A 3 | E 1 | M 4 | graubraun |
| A 4 | E 1 | M 5 | dunkelrubin |
| A 5 | E 1 | M 7 | dunkelrubin |
| A 6 | E 1 | M 8 | aubergine |
| A 7 | E 1 | M 9 | dunkelpurpur |
| A 8 | E 1 | M 11 | graubraun |
| A 9 | E 1 | M 12 | dunkelviolett |
| A 10 | E 1 | M 13 | dunkelviolett |
| A 11 | E 1 | M 14 | schwarzblau |
| A 12 | E 1 | M 15 | schwarzblau |
| A 13 | E 1 | M 16 | violettbraun |
| A 14 | E 1 | M 17 | dunkelpurpur |
| A 15 | E 1 | M 18 | schwarzblau |
| A 16 | E 1 | M 19 | violettbraun |

...

| | | | |
|---|---|---|---|
| A 17 | E 1 | M 20 | violettbraun |
| A 18 | E 2 | M 1 | schwarzblau |
| A 19 | E 2 | M 4 | dunkelviolett |
| A 20 | E 2 | M 9 | dunkelviolett |
| A 21 | E 3 | M 2 | grauviolett |
| A 22 | E 3 | M 6 | hellbraun |
| A 23 | E 3 | M 9 | rotbraun |
| A 24 | E 3 | M 15 | violett |
| A 25 | E 4 | M 6 | olivbraun |
| A 26 | E 4 | M 8 | rotbraun |
| A 27 | E 4 | M 9 | violettbraun |
| A 28 | E 4 | M 10 | dunkelviolett |
| A 29 | E 4 | M 21 | aubergine |
| A 30 | E 4 | M 22 | dunkelbraun |
| A 31 | E 4 | M 25 | braun |
| A 32 | E 4 | M 26 | schokoladenbraun |
| A 33 | E 4 | M 28 | dunkelbraun |
| A 34 | E 5 | M 1 | dunkelblau |
| A 35 | E 5 | M 3 | olivbraun |
| A 36 | E 5 | M 4 | teakholzfarbig |
| A 37 | E 5 | M 9 | dunkelpurpur |
| A 38 | E 5 | M 10 | blauviolett |
| A 39 | E 5 | M 15 | schwarzblau |
| A 40 | E 5 | M 22 | dunkelbraun |
| A 41 | E 5 | M 23 | dunkelviolett |
| A 42 | E 5 | M 24 | graubraun |
| A 43 | E 5 | M 27 | rußbraun |
| A 44 | E 5 | M 29 | dunkelbraun |

. . .

## Patentansprüche

1. Haarfärbemittel, enthaltend Oxidationshaarfarbstoffvorprodukte in einem kosmetischen Träger, dadurch gekennzeichnet, daß als Oxidationsfarbstoffvorprodukte N-Benzyl-p-phenylendiamin oder dessen Derivate der allgemeinen Formel I

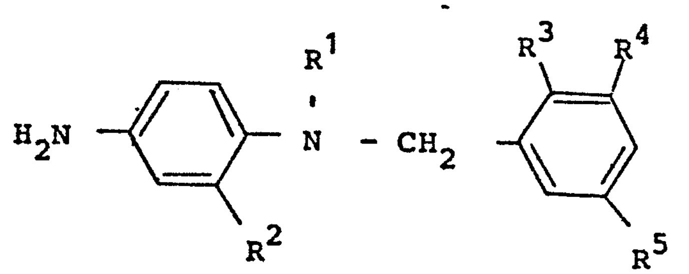

(I)

in der $R^1$ Wasserstoff oder eine Alkylgruppe mit 1 - 4 C-Atomen, $R^2$ Wasserstoff, Chlor oder eine Alkylgruppe mit 1 - 4 C-Atomen, $R^3$ Wasserstoff oder eine Hydroxylgruppe und $R^4$ und $R^5$ unabhängig voneinander Wasserstoff oder Chlor bedeuten, oder deren Salz und die in Oxidationsfärbemitteln üblichen Kuppler- und/oder Entwicklersubstanzen enthalten sind.

2. Haarfärbemittel nach Anspruch 1, dadurch gekennzeichnet, daß als Oxidationsfarbstoffvorprodukte Verbindungen der allgemeinen Formel I enthalten sind, in welchen die Substituenten die folgende Bedeutung haben:

| | | | | | |
|---|---|---|---|---|---|
| a: | $R^1$ = H, | $R^2$ = H, | $R^3$ = H, | $R^4$ = H, | $R^5$ = H |
| b: | $R^1$ = $CH_3$, | $R^2$ = H, | $R^3$ = H, | $R^4$ = H, | $R^5$ = H |
| c: | $R^1$ = H, | $R^2$ = Cl, | $R^3$ = H, | $R^4$ = H, | $R^5$ = H |
| d: | $R^1$ = H, | $R^2$ = H, | $R^3$ = OH, | $R^4$ = Cl, | $R^5$ = Cl |
| e: | $R^1$ = H, | $R^2$ = $CH_3$, | $R^3$ = OH, | $R^4$ = H, | $R^5$ = H |

. . .

3. Haarfärbemittel nach Ansoruch 1 oder 2, dadurch gekennzeichnet, daß zusätzlich direktziehende Haarfarbstoffe enthalten sind.

4. Haarfärbemittel nach Anspruch 1 - 3, dadurch gekennzeichnet, daß N-Benzyl-p-phenylendiamin oder dessen Derivate der allgemeinen Formel I in einer Menge von 0,05 - 10 Millimol pro 100 g enthalten sind.

. . .